# EUROPEAN PATENT SPECIFICATION

(11) **EP 1 407 731 B1**
(45) Date of publication and mention of the grant of the patent: **21.06.2006**
(21) Application number: 04000898.9
(22) Date of filing: 11.04.1996
(51) Int. Cl.: A61F 5/448

(54) **Ostomy coupling**
Ostomiekupplung
Accouplement d'ostomie

(30) Priority: 13.04.1995 GB 9507666; 30.08.1995 GB 9517666
(43) Date of publication of application: 14.04.2004
(62) Divisional of application: 96302557.2
(73) Proprietor: Bristol-Myers Squibb Company, New York, N.Y. 10154 (US)
(72) Inventor: Steer, Peter Leslie, Kingscote East Grinstead Sussex RH10 4JZ (GB); Hollands, Keith G. M., Sompting Sussex (GB)
(74) Representative: Holmes, Miles Keeton

(56) References cited:
- EP-A- 0 286 501
- EP-A- 0 381 393
- GB-A- 2 289 221

## Description

This invention relates to an ostomy coupling.

Ostomy couplings are used to connect and disconnect a bag for receiving a stomal discharge to and from a medical grade adhesive pad which is applied to the peristomal area of the skin of the wearer. Many designs of ostomy coupling are known. One which has enjoyed considerable commercial success is described and claimed in U.K. Patent No. 1,571,657.

An ostomy coupling in which unlocking of two coupling parts is achieved by deforming a ring in the form of a closed loop is disclosed in our U.K. Patent Application No. 9409037.0, filed 6 May 1994.

It has been proposed by Kubo, in Japanese Utility Model No. 62-11610, published February 1985, that an ostomy device should have a double female ring structure which can interengage with a male ring. The male ring may be on the bag and the female ring on a skin-attachable adhesive pad, or vice-versa. The outer ring on the female ring is circular and flexible and has a pair of inwardly-extending catches at opposite ends of a diameter. By pressing on two diametrically extending lugs, whose diameter is substantially at right angles to the diameter joining the catches, the outer female ring is deformed so that the catches are caused to move radially outwardly, so permitting separation of the two coupling parts.

This arrangement, though perhaps operable in theory, has serious disadvantages in practice, for example (i) to connect or disconnect it is necessary to hold the coupling at four places, approximately spaced at 90° intervals around the periphery, (ii) pressing on two diametrically opposed regions will tend to bend the coupling out of its normal plane and the forces applied may easily cause the body side pad to be partially (or wholly) detached from the skin of the wearer, also the need to press in both ends of the diameter fully, and simultaneously, means that releasing the bag-side coupling is subject to uncertainty, (iii) the repeated attachment and withdrawal of the bag-side coupling part will cause the o-ring (provided to prevent escape of excreted matter between the male and female rings) to become worn, so compromising its sealing qualities with potentially highly embarrassing and undesirable results, (iv) the wearer may find it difficult to determine whether or not the two coupling parts are properly engaged, (v) the accuracy and forces needed for manipulation to connect or disconnect will be well beyond the capability of an infirm, confused, elderly or impatient wearer; (vi) it is hard to be sure that the appliance is properly locked; and (vii) in the case of large sizes, the old and infirm will find it physically difficult to span with their hand and push in diametrally opposed regions of the ring.

In U.S. Patent No. 5180377 there is disclosed an ostomy coupling in which a bag-side coupling attached to an ostomy pouch can be held onto to a body-side member by a third member which is substantially circular, deformable, and clamps or grips the bag side coupling when it is tightened. In practice, the diameter of the third member is reduced by operation of a mechanical control mechanism, some of whose parts are attached to the third member. While it may be true, as the patentee states, that a sealed mechanical connection of bag- and body-side couplings can be achieved using such a mechanism, the overall construction is relatively complex, which of course affects manufacturing costs.

PCT published Applications Nos. WO91/01118 and WO91/01119 relate to an ostomy coupling and to a ring for locking such a coupling. The ring has two free ends which are manually pulled together to tighten the ring circumferentially around bag- and body-side coupling parts whose structure is designed in various ways so that they can inter-engage. This results in a variety of relatively complex designs, likely to be costly to manufacture. Manipulation of such a coupling will potentially give rise to difficulties for infirm, elderly, or non-dexterous persons.

EP-A-0680736 discloses under Article 54(3) EPC an ostomy coupling comprising a first coupling element and a second coupling element. The former is of substantially channel shape as seen in cross-section. The second coupling element is of complementary closed loop form and has an extending rib extending upwardly from a flange. The rib carries a peripheral deflectable seal. The two coupling elements are connectible by a deformable closed ring which is made captive to one coupling element at one circumferential region thereof and which has a member constrained to move in a radial direction which, when so moved, causes a deformation of the ring whereby tabs thereon are shifted clear of overlap with a rim portion of the other coupling element, so permitting separation of the two coupling elements. Pressing the two separate coupling elements together axially expands the deformable closed ring to permit the coupling members to come into mutual fastened engagement.

EP-A-0286501 describes a connection assembly for a drainage appliance, one portion attaching to the body of the patient, the other portion attached to the drainage collection bag. The connection is said to allow easy removal of the bag yet assure an easily lockable joint including a continuous seal between the two portions when connected together. The two portions fit together and resilient snap-fit elements create an initially sealed relationship. A rotating closed locking ring on the body portion can be rotated to lock the snap-fit elements in a non-resilient condition and locks the two portions together while still permitting relative rotational motion therebetween. The bag portion can be easily removed when the locking ring is moved to an unlocked position, thereby releasing the snap-fit elements.

EP-A-0381393 describes a coupling element system for an ostomy bag including a bag-side coupling element having a stomal orifice surrounded by a channel shaped member, a body-side coupling element having a stomal orifice surrounded by a rib-like member which can make snap-fit engagement with the channel shaped member, and a locking member. The latter member has radially inwardly deformable arms and is rotatable relative to the body-side coupling. The deformable arms are shaped and positioned so that upon rotation of the locking member in one rotary direction relative to the body-side coupling element they are deformed radially inwardly and over-lie a portion of the bag-side coupling element. The locking member may be generally annular with the arms arcuate in shape each with a blade portion and a leg portion which connects the blade portion to the remainder of the locking member.

WO-A-9101118, US-A-5322522 and US-A-5322523 describe an ostomy coupling comprising a patient part, a bag part and a locking ring for mutually retaining these two parts. The locking ring has a first locking mechanism with two positions of the locking ring, in which the coupled parts are mutually loosely connected and mutually locked, respectively. The locking ring furthermore has a second locking mechanism being independent of the first locking mechanism and adapted for retaining and releasing, respectively, the locking ring relatively to one of the parts.

It is an aim of this invention to provide improved designs of ostomy couplings which embody a springy or resilient split ring as a locking ring. That is, the ring is generally circular in form but has a single gap therein, subtending a small angle, e.g. 5 to 15°.

The present invention is defined in the claims.

The invention will be better understood from the following non-limiting description of an example thereof given with reference to the accompanying drawings in which:-
Figure 1 is a cross-sectional view on the plane B-B of Figure 2 of an embodiment of the invention, showing a first or body-side coupling member, in plan view;
Figure 2 is an elevation, but with the left-hand half partly in section, of the first coupling member shown in Figure 1;
Figure 3 is a plan view of a split locking ring forming part of an ostomy coupling according to the invention;
Figure 4 is a side elevation of the ring looking in the direction "E" of Figure 3;
Figure 5 is a cross-sectional view, on the plane C-C of Figure 3, of the locking ring shown in Figure 3;
Figures 6 and 7 are respectively plan and side elevation views, the latter partly in cross-section of a second coupling member (which will normally be the bag-side member; and
Figure 8 is a view in axial cross-section on an enlarged scale, showing the parts of the coupling in assembled condition with the locking ring being shown in its locked position.

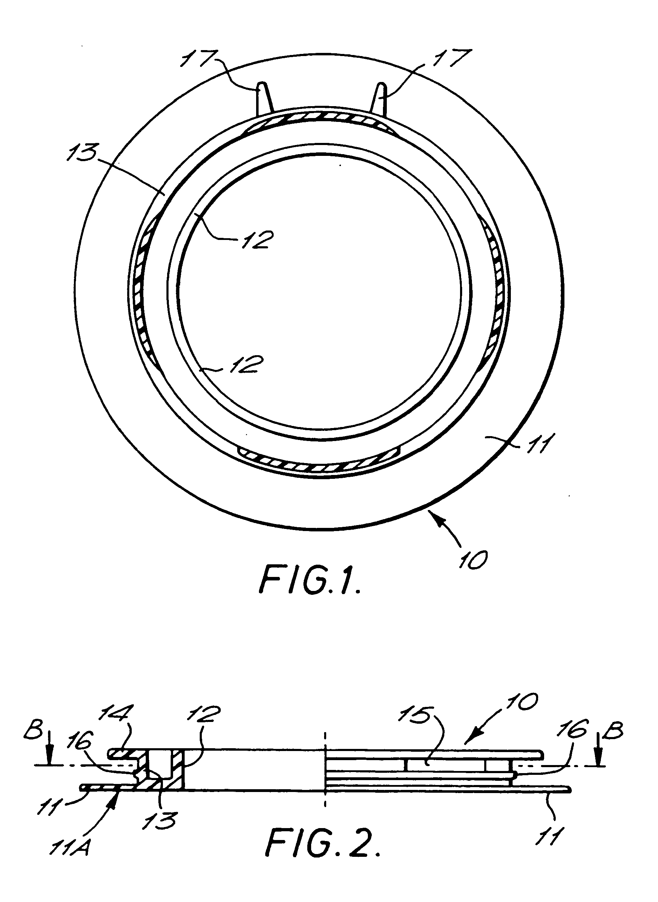

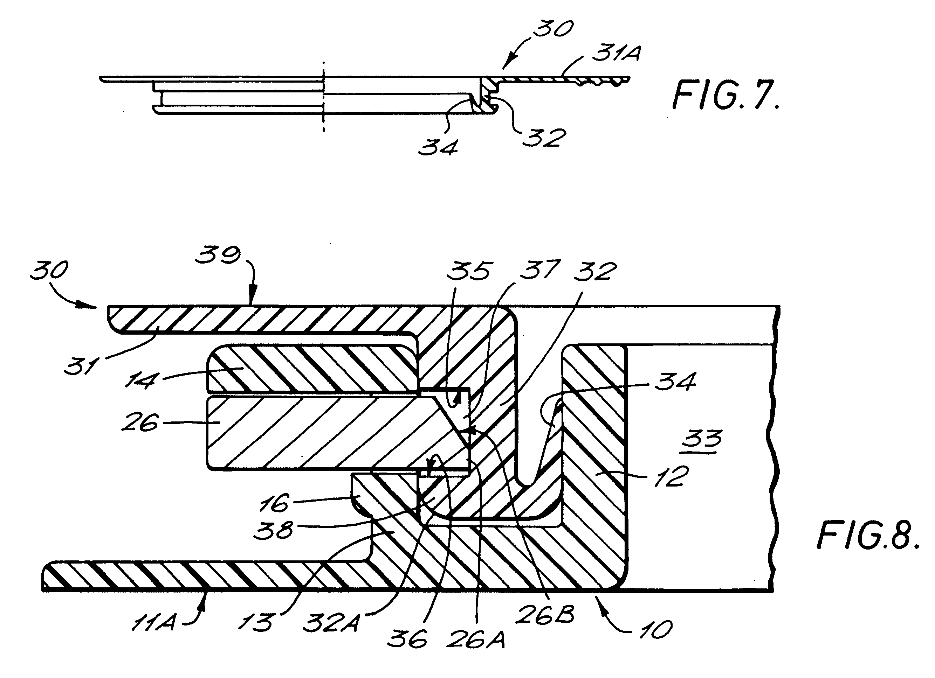

In accordance with a preferred embodiment of this invention, there is now described a first coupling member 10 which will normally be a body-side coupling member. This comprises a lower flange 11, having a surface 11A to which may be attached a medical grade adhesive pad. Such medical grade adhesive pads are known, and are used to attach the ostomy appliance to the skin of the wearer in the peristomal region. The pad comprises a base which is preferably a thin film of polymeric material such as polyethylene and an adhesive layer situated on the rear surface of the base. Such an adhesive layer is preferably formed as a homogeneous blend of one or more pressure-sensitive viscous or elastomeric materials having intermittently dispersed therein one or more water-soluble or swellable hydrocolloid gums and may also include one or more thermoplastic elastomers and/or one or more swellable cohesive strengthening agents. The first coupling member 10 also has a radially inner wall 12, and a radially outer wall 13. Together these walls, with flange 11, define an annular channel, there being a radially outwardly extending flange 14, substantially parallel to the flange 11, and attached to or integral with the annular channel. The channel is best seen in the left-hand portion of Figure 2. The wall 13 has therein a plurality of apertures 15, four in number, which are located to permit passage of respective locking blades 26 through the apertures, as seen in Figure 8. A radially outstanding rim 16, of shallow radius, is disposed on the outer surface of the wall 13. As seen in Figure 1, a pair of stops 17 are located (in the position when the ostomy coupling member is being worn) at the upper region of the member and at respective positions such that the angle subtended between them is approximately 30°. Of course other suitable angles may be used instead of 30°, as will be understood by one skilled in the art.

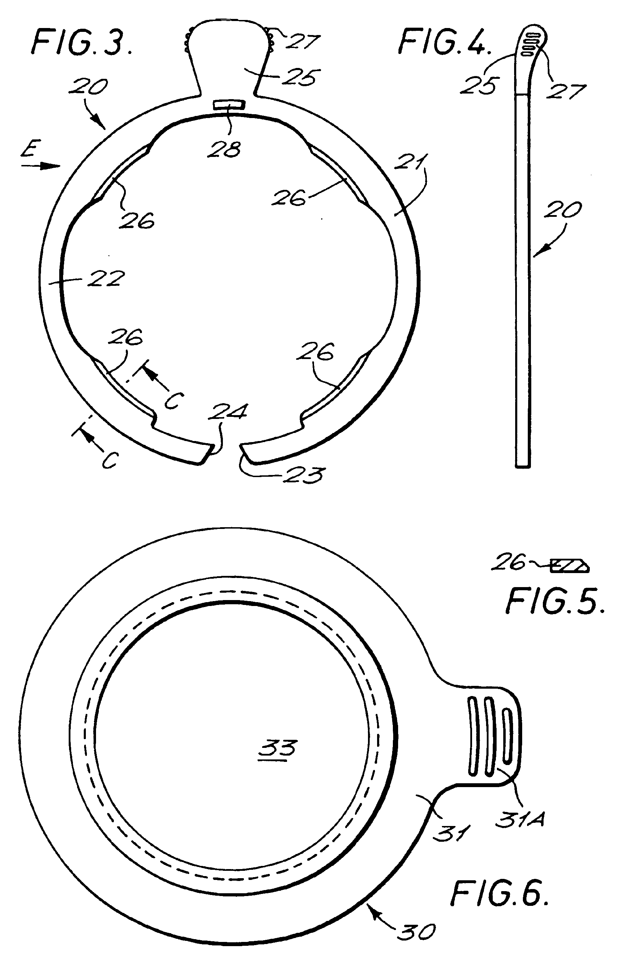

Figures 3, 4 and 5 illustrate a preferred form of locking ring 20 for use in an ostomy appliance according to the invention. This ring is a split ring, that is, it comprises a pair of limbs 21 and 22 having free ends 23 and 24, these free ends in the normal rest or unstressed position of the ring being spaced from each other by a distance that subtends an angle of approximately 10°. Integral with each limb 21 of the locking ring, are locking blades 26 which extend generally radially inwardly. At the top of the locking ring 20, a handle 25 having gripping ribs 27 is provided to enable the wearer to rotate the locking ring relative to the coupling member 10. A stud 28 projects from the split ring 10 into the gap between the stops 17 (when the parts are assembled).

The third element of the ostomy appliance is the second coupling ring 30 which normally will be the bag-side coupling member. This comprises a radially extending flange 31 integral with a substantially cylindrical wall 32 which surrounds a stomal orifice 33. An ostomy bag or pouch is attached in any suitable manner to a surface 39 of the ring 30. Extending inwardly from one side of the wall 32 is a flexible resilient sealing strip 34 whose main function is to prevent leakage of liquids or other waste material through the coupling when the bag-side coupling member is attached to the body-side coupling member and locked thereon, due to the positioning of the ring 20. Strip 34 also takes up moulding tolerances. On the radially external surface of the wall 32, a groove 37 is provided, bounded by surfaces 35 and 36, there being a rim 38 which serves a locking function in cooperation with the locking blade 26, preventing the coupling members 10 and 30 from being separated from each other in an axial direction. The rim 16 also assists in guiding the locking blades 26 into and out of the groove 13, when the locking ring 20 is moved in a rotary direction. This rotary movement is limited in either rotary direction by the abutment of a stop 28 on the locking ring 20 with respective stops 17 on the first coupling member 10. A grip tab 31A is made in one piece with the flange 31 of the second coupling member 30. Grip tabs are well known in ostomy couplings and grip tab 31A forms no part of the novelty of the present invention.

In operation, given that the coupling members and the locking ring are assembled together as shown in Figure 8, and assuming that an ostomate wishes to remove the second coupling member and the pouch attached thereto, he or she would then grasp the handle 25 between finger and thumb and shift it to cause the locking ring to make a rotating movement, this movement shifting the stud 28 from engagement with one of the stops 17 and moving it into engagement with the other of the stops 17. As a consequence the locking ring is rotated relative to the first and second coupling members which remain non-rotative. The blades 26, due to their curved surfaces 26A, are forced radially outwardly of the respective apertures 15. This causes the blade 26 as seen in Figure 8 to move horizontally in a direction towards the right as seen in the Figure, until the inner edge 26A of that blade is shifted to be clear of the annular channel occupied by the second coupling member 30. The coupling member 30 and the pouch attached thereto may then be axially withdrawn from the first coupling member 10.

The ostomate may then if he wishes return the handle 25 to its original position but if this is not done, the chamfer surface 26B in combination with the curved surface 32A enables a new second coupling ring to be axially pushed into the first coupling ring against the resilience of the locking ring 20.

It will be appreciated that variations may be made without departing from the invention. For example, while reference has been to circular first and second coupling members, coupling members of other closed loop forms, e.g. oval, could be employed subject to certain adjustments to the design. While the first coupling member has been referred to as a body-side coupling member and the second as a bag-side coupling member, these parts could be reversed if desired. That is to say, coupling member 10 could be attached to a pouch and coupling member 30 could be attached to a pad of medical grade adhesive on the skin of the ostomate. While four locking blades 26 have been shown in the above drawings, a different number of locking blades, and a corresponding different number of apertures, could be employed instead. Other suitable detents or movement limiting members could be employed instead of the stop 17 and stud 28, without departing from the invention.

## Claims

1. An ostomy coupling in which first and second coupling members (10, 30) are securable together by a springy flexible split locking ring (20) carried on the first coupling member (10), and in which a plurality of tabs (26), arranged on limbs of the locking ring, can be withdrawn generally radially outwardly by movement of the locking ring (20) from a lock position to an unlock position to permit separation of the two coupling members (10, 30);
wherein the first and second coupling members (10, 30) and the locking ring (20) are configured to permit the first and second coupling members (10, 30) to be pressed together into locking engagement against the resilience of the locking ring (20) when the locking ring (20) is in the lock position, the locking ring (20) being configured to expand temporarily under the action of the coupling members (10, 30) being pressed together.

2. An ostomy coupling according to claim 1, wherein when the first and second coupling members (10, 30) are pressed together, the second coupling member (30) bears on the tabs (26) to force the tabs (26) outwardly against the resilience of the locking ring (20).

3. An ostomy coupling according to claim 2, wherein the locking ring (20) urges the tabs (26) generally radially inwardly to secure the coupling members (10, 30) together when the second coupling member (30) reaches a fully coupled position relative to the first coupling member (10).

4. An ostomy coupling according to any preceding claim, wherein each tab (26) has a chamfer surface (26B) engageable by the second coupling member (30) when the first and second coupling members are pressed together against the resilience of the locking ring (20).

5. An ostomy coupling according to any preceding claim, wherein the second coupling member (30) comprises a rounded profile (32a) for contacting and displacing the tabs (26) radially outwardly when the first and second coupling members (10, 30) are pressed together against the resilience of the locking ring (20).

6. An ostomy coupling according to any preceding claim, wherein the second coupling member (30) comprises a groove for interlocking engagement by the tabs (26).

7. An ostomy coupling according to any preceding claim, wherein said movement of the locking ring (20) for withdrawing the tabs is movement in a rotary direction.

8. An ostomy coupling according to any preceding claim, wherein each tab (26) comprises a chamfer surface (26A) on one face, and an non-chamfered surface on an opposite face.

9. An ostomy coupling according to any preceding claim, wherein the first coupling member (10) comprises a plurality of apertures (13) through which the tabs (26) project when the locking ring (20) is in the lock position.

10. An ostomy coupling according to claim 9, wherein each tab comprises a curved surface for engaging an edge of its associated aperture, for causing the tab to be withdrawn by said movement of the locking ring.

11. An ostomy coupling member (10) for use with a complementary coupling member, said ostomy coupling member (10) carrying a springy flexible split locking ring (20) having tabs (26) arranged on limbs of the locking ring (20), the tabs (26) projecting into a channel for receiving a portion of a said complementary coupling member, in use, for securing the two coupling members together, and the tabs (26) being withdrawable generally radially outwardly by movement of the locking ring (20) from a lock position to an unlock position,
wherein the tabs (20) have a chamfer surface (26A) configured such that, in use, when the ostomy coupling member and a said complementary coupling member are pressed together when the locking member (20) is in the lock position, the tabs are displaced radially outwardly against the resilience of the locking ring (20) thereby to permit the ostomy coupling member (10) and a said complementary coupling member to be pressed together into locking engagement.

## Patentansprüche

1. Ostomiekupplung, in der erste und zweite Kupplungsglieder (10, 30) miteinander verriegelbar sind durch einen federnden flexiblen Split-Schließring (20), der von dem ersten Kupplungsglied (10) gehalten wird, und in der eine Anzahl von Streifen (26), die auf Schenkeln des Schließringes angeordnet sind, durch ein Bewegen des Schließringes (20) aus einer Schließstellung in eine Nichtschließstellung weitgehend radial nach außen herausgezogen werden können, um ein Abtrennen der beiden Kupplungsglieder (10, 30) zu ermöglichen,
wobei die ersten und zweiten Kupplungsglieder (10, 30) und der Schließring (20) so gestaltet sind, dass sie gegen die Federwirkung des Schließringes (20) ein Zusammendrücken der ersten und zweiten Kupplungsglieder (10, 30) in eine Schließverbindung ermöglichen, wenn der Schließring (20) in der Schließstellung ist, wobei der Schließring (20) so gestaltet ist, dass er sich vorübergehend unter der Einwirkung der Kupplungsglieder (10, 30), die zusammengedrückt werden, ausdehnt.

2. Ostomiekupplung nach Anspruch 1, wobei dann, wenn die ersten und zweiten Kupplungsglieder (10, 30) zusammengedrückt werden, das zweite Kupplungsglied (30) auf die Streifen (26) drückt, um die Streifen (26) gegen die Federwirkung des Schließringes (20) nach außen zu zwingen.

3. Ostomiekupplung nach Anspruch 2, wobei der Schließring (20) die Streifen (26) weitgehend radial nach innen drückt, um die Kupplungsglieder (10, 30) miteinander zu verriegeln, wenn das zweite Kupplungsglied (30) eine vollständig gekoppelte Stellung bezüglich des ersten Kupplungsgliedes (10) erreicht.

4. Ostomiekupplung nach einem der vorhergehenden Ansprüche, wobei jeder Streifen (26) eine abgeschrägte Oberfläche (26B) aufweist, welche durch das zweite Kupplungsglied (30) erfasst werden kann, wenn das erste und zweite Kupplungsglied gegen die Federwirkung des Schließringes (20) zusammengedrückt werden.

5. Ostomiekupplung nach einem der vorhergehenden Ansprüche, wobei das zweite Kupplungsglied (30) ein abgerundetes Profil (32A) für das Kontaktieren und Verschieben der Streifen (26) radial nach außen aufweist, wenn das erste und zweite Kupplungsglied (10, 30) gegen die Federwirkung des Schließringes (20) zusammengedrückt werden.

6. Ostomiekupplung nach einem der vorhergehenden Ansprüche, wobei das zweite Kupplungsglied (30) eine Kerbe für den Schließeingriff durch die Streifen (26) aufweist.

7. Ostomiekupplung nach einem der vorhergehenden Ansprüche, wobei die Bewegung des Schließringes (20) zum Herausziehen der Streifen eine Bewegung in einer Drehrichtung ist.

8. Ostomiekupplung nach einem der vorhergehenden Ansprüche, wobei jeder Streifen (26) auf der einen Fläche eine abgeschrägte Oberfläche (26A) und auf einer gegenüberliegenden Fläche eine nicht abgeschrägte Oberfläche ausweist.

9. Ostomiekupplung nach einem der vorhergehenden Ansprüche, wobei das erste Kupplungsglied (10) eine Anzahl von Öffnungen (13) aufweist, durch welche die Streifen (26) herausragen, wenn der Schließring (20) in der Schließstellung ist.

10. Ostomiekupplung nach Anspruch 9, wobei jeder Streifen eine gekrümmte Oberfläche zum Erfassen einer Flanke seiner zugehörigen Öffnung aufweist, um durch die Bewegung des Schließringes ein Zurückziehen der Streifen zu bewirken.

11. Ostomiekupplung (10) zur Verwendung mit einem ergänzenden Kupplungsglied, wobei das Ostomie-Kupplungsglied (10) einen federnden flexiblen Split-Schließring (20) trägt, der auf Schenkeln des Schließringes (20) angeordnete Streifen (26) aufweist, die Streifen (26) in einen Kanal für die Aufnahme eines Teils des ergänzenden Kupplungsgliedes hineinragen, um beim Einsatz die beiden Kupplungsglieder miteinander zu verriegeln, und die Streifen (26) durch Bewegung des Schließringes (20) aus einer Schließstellung in eine Nichtschließstellung weitgehend radial nach außen herausgezogen werden können,
wobei die Streifen (20) eine abgeschrägte Oberfläche (26A) aufweisen, die derart gestaltet ist, dass beim Einsatz, wenn das Ostomie-Kupplungsglied und das ergänzende Kupplungsglied zusammengedrückt sind, wenn das Schließglied (20) in der Schließlage ist, die Streifen gegen die Federwirkung des Schließringes (20) radial nach außen verschoben werden, wodurch es ermöglicht wird, das Ostomie-Kupplungsglied (10) und das ergänzende Kupplungsglied in eine Schließverbindung zusammenzudrücken.

## Revendications

1. Accouplement de stomie dans lequel des premier et second éléments d'accouplement (10, 30) peuvent être fixés ensemble par un anneau de verrouillage fendu élastique et souple (20) porté sur le premier élément d'accouplement (10), et dans lequel une pluralité d'ergots (26) agencés sur les membres de l'anneau de verrouillage peuvent être retirés généralement radialement vers l'extérieur par le déplacement de l'anneau de verrouillage (20) d'une position verrouillée à une position déverrouillée afin de permettre la séparation des deux éléments d'accouplement (10, 30),
dans lequel les premier et second éléments d'accouplement (10, 30) et l'anneau de verrouillage (20) sont configurés afin de permettre aux premier et second éléments d'accouplement (10, 30) d'être pressés l'un contre l'autre jusqu'en engagement de verrouillage en s'opposant à l'élasticité de l'anneau de verrouillage (20) lorsque l'anneau de verrouillage (20) est dans la position verrouillée, l'anneau de verrouillage (20) étant configuré de façon à s'agrandir momentanément sous l'action des éléments d'accouplement (10, 30) qui sont pressés l'un contre l'autre.

2. Accouplement de stomie selon la revendication 1, dans lequel lorsque les premier et second éléments d'accouplement (10, 30) sont pressés l'un contre l'autre, le second élément d'accouplement (30) porte sur les ergots (26) afin de forcer les ergots (26) vers l'extérieur en s'opposant à l'élasticité de l'anneau de verrouillage (20).

3. Accouplement de stomie selon la revendication 2, dans lequel l'anneau de verrouillage (20) sollicite les ergots (26) généralement radialement vers l'intérieur afin de fixer les éléments d'accouplement (10, 30) ensemble lorsque le second élément d'accouplement (30) atteint une position totalement couplée par rapport au premier élément d'accouplement (10).

4. Accouplement de stomie selon l'une quelconque des revendications précédentes, dans lequel chaque ergot (26) possède une surface de chanfrein (26B) qui peut être engagée par le second élément d'accouplement (30) lorsque les premier et second éléments d'accouplement sont pressés l'un contre l'autre en s'opposant à l'élasticité de l'anneau de verrouillage (20).

5. Accouplement de stomie selon l'une quelconque des revendications précédentes, dans lequel le second élément d'accouplement (30) comprend un profil arrondi (32a) pour venir en contact et déplacer les ergots (26) radialement vers l'extérieur lorsque les premier et second éléments d'accouplement (10, 30) sont pressés l'un contre l'autre en s'opposant à l'élasticité de l'anneau de verrouillage (20).

6. Accouplement de stomie selon l'une quelconque des revendications précédentes, dans lequel le second élément d'accouplement (30) comprend une rainure destinée à un engagement par verrouillage mutuel par les ergots (26).

7. Accouplement de stomie selon l'une quelconque des revendications précédentes, dans lequel le déplacement de l'anneau de verrouillage (20) en vue de retirer les ergots est un déplacement dans le sens d'une rotation.

8. Accouplement de stomie selon l'une quelconque des revendications précédentes, dans lequel chaque ergot (26) comprend une surface de chanfrein (26A) sur une face, et une surface non chanfreinée sur la face opposée.

9. Accouplement de stomie selon l'une quelconque des revendications précédentes, dans lequel le premier élément d'accouplement (10) comprend une pluralité d'ouvertures (13) au travers desquelles les ergots (26) dépassent lorsque l'anneau de verrouillage (20) est dans la position verrouillée.

10. Accouplement de stomie selon la revendication 9, dans lequel chaque ergot comprend une surface incurvée destinée à un engagement d'un bord de son ouverture associée, afin d'amener l'ergot à être retiré par ledit déplacement de l'anneau de verrouillage.

11. Elément d'accouplement de stomie (10) destiné à une utilisation avec un élément d'accouplement complémentaire, ledit élément d'accouplement de stomie (10) portant un anneau de verrouillage fendu élastique et souple (20) ayant des ergots (26) agencés sur les membres de l'anneau de verrouillage (20), les ergots (26) dépassant dans un canal afin de recevoir une partie d'un dit élément d'accouplement complémentaire, en utilisation, afin de fixer les deux éléments d'accouplement ensemble, et les ergots (26) pouvant être retirés généralement radialement vers l'extérieur par le déplacement de l'anneau de verrouillage (20) d'une position verrouillée à une position déverrouillée,
dans lequel les ergots (26) ont une surface de chanfrein (26A) configurée de façon à ce que, en utilisation, lorsque l'élément d'accouplement de stomie et un dit élément d'accouplement complémentaire sont pressés l'un contre l'autre lorsque l'anneau de verrouillage (20) se trouve dans une position verrouillée, les ergots sont déplacés radialement vers l'extérieur en s'opposant à l'élasticité de l'anneau de verrouillage (20) en permettant ainsi à l'élément d'accouplement de stomie (10) et à un dit élément d'accouplement complémentaire d'être pressés l'un contre l'autre en engagement de verrouillage.
